# FASCICULE DE BREVET EUROPEEN

(11) **EP 2 627 667 B1**
(45) Date de publication et mention de la délivrance du brevet: **13.04.2016**
(21) Numéro de dépôt: 11776578.4
(22) Date de dépôt: 14.10.2011
(51) Int. Cl.: C07K 14/415, C12N 15/82

(54) **OBTENTION DE PLANTES AYANT UNE TOLÉRANCE AMÉLIORÉE À UN DÉFICIT HYDRIQUE**
HERSTELLUNG VON PFLANZEN MIT VERBESSERTER TOLERANZ GEGENÜBER WASSERMANGEL
PRODUCTION OF PLANTS HAVING IMPROVED WATER-DEFICIT TOLERANCE

(30) Priorité: 15.10.2010 FR 1004059
(43) Date de publication de la demande: 21.08.2013
(73) Titulaire: Genoplante-Valor, 75015 Paris (FR)
(72) Inventeur: ROUSTER, Jacques, 63730 Mirefleurs (FR); SALLAUD, Christophe, 63110 Beaumont (FR); COURSOL, Sylvie, 75012 Paris (FR); ZIVY, Michel, 75010 Paris (FR); VIRLOUVET, Laetitia, 91360 EPINAY SUR ORGE (FR); WELCKER, Claude, 34980 Montferrier-sur-Lez (FR)
(74) Mandataire: Gevers & Orès
(86) Numéro de dépôt international: PCT/IB2011/054570
(87) Numéro de publication internationale: WO 2012/049663

(56) Documents cités:
- WO-A2-2009/056566
- WO-A2-2009/127441

### SEQUENCE LISTING

<110> GENOPLANTE-VALOR
   ROUSTER, Jacques
   SALLAUD, Christophe
   COURSOL, Sylvie
   ZIVY, Michel
   VIRLOUVET, Laetitia
   WELCKER, Claude
<120> OBTENTION DE PLANTES AYANT UNE TOLERANCE AMELIOREE A UN DEFICIT HYDRIQUE
<130> MJP/XRN/cc-F1516/38/PCT
<150> FR 1004059
   <151> 2010-10-15
<160> 5
<170> PatentIn version 3.5
<210> 1
   <211> 275
   <212> PRT
   <213> Zea mays
<400> 1
   Met Gly Arg Ser Pro Cys Cys Glu Lys Ala His Thr Asn Lys Gly Ala
   1               5                   10                  15
   Trp Thr Lys Glu Glu Asp Glu Arg Leu Val Ala His Ile Arg Ala His
               20                  25                  30
   Gly Glu Gly Cys Trp Arg Ser Leu Pro Lys Ala Ala Gly Leu Leu Arg
           35                  40                  45
   Cys Gly Lys Ser Cys Arg Leu Arg Trp Ile Asn Tyr Leu Arg Pro Asp
       50                  55                  60
   Leu Lys Arg Gly Asn Phe Thr Glu Glu Glu Asp Glu Leu Ile Val Lys
   65                  70                  75                  80
   Leu His Ser Val Leu Gly Asn Lys Trp Ser Leu Ile Ala Gly Arg Leu
                   85                  90                  95
   Pro Gly Arg Thr Asp Asn Glu Ile Lys Asn Tyr Trp Asn Thr His Ile
               100                 105                 110
   Arg Arg Lys Leu Leu Ser Arg Gly Ile Asp Pro Val Thr His Arg Pro
           115                 120                 125
   Val Thr Glu His His Ala Ser Asn Ile Thr Ile Ser Phe Glu Thr Glu
       130                 135                 140
   Val Ala Ala Ala Ala Arg Asp Asp Lys Lys Gly Ala Val Phe Arg Leu
   145 150 155 160
   Glu Asp Glu Glu Glu Glu Glu Arg Asn Lys Ala Thr Met Val Val Gly
   165 170 175
   Arg Asp Arg Gln Ser Gln Ser His Ser His Ser His Pro Ala Gly Glu
   180 185 190
   Trp Gly Gln Gly Lys Arg Pro Leu Lys Cys Pro Asp Leu Asn Leu Asp
   195 200 205
   Leu Cys Ile Ser Pro Pro Cys Gln Glu Glu Glu Glu Met Glu Glu Ala
   210 215 220
   Ala Met Arg Val Arg Pro Ala Val Lys Arg Glu Ala Gly Leu Cys Phe
   225 230 235 240
   Gly Cys Ser Leu Gly Leu Pro Arg Thr Ala Asp Cys Lys Cys Ser Ser
   245 250 255
   Ser Ser Phe Leu Gly Leu Arg Thr Ala Met Leu Asp Phe Arg Ser Leu
   260 265 270
   Glu Met Lys
   275
<210> 2
   <211> 53
   <212> PRT
   <213> Zea mays
<220>
   <221> misc_feature
   <222> (1)..(5)
   <223> Xaa can be any naturally occurring amino acid
<220>
   <221> misc_feature
   <222> (7)..(25)
   <223> Xaa can be any naturally occurring amino acid
<220>
   <221> misc_feature
   <222> (27)..(45)
   <223> Xaa can be any naturally occurring amino acid
<220>
   <221> misc_feature
   <222> (47)..(53)
   <223> Xaa can be any naturally occurring amino acid
<400> 2
   Xaa Xaa Xaa Xaa Xaa Trp Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Trp Xaa Xaa Xaa Xaa Xaa Xaa
               20                  25                  30
   Xaa Xaa Xaa Xaa Xaa Xaa Xaa Xaa Xaa Xaa Xaa Xaa Xaa Trp Xaa Xaa
           35                  40                  45
   Xaa Xaa Xaa Xaa Xaa
       50
<210> 3
   <211> 51
   <212> PRT
   <213> Zea mays
<220>
   <221> misc_feature
   <222> (1)..(24)
   <223> Xaa can be any naturally occurring amino acid
<220>
   <221> misc_feature
   <222> (26)..(43)
   <223> Xaa can be any naturally occurring amino acid
<220>
   <221> misc_feature
   <222> (45)..(51)
   <223> Xaa can be any naturally occurring amino acid
<400> 3
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Trp Xaa Xaa Xaa Xaa Xaa Xaa Xaa
               20                  25                  30
   Xaa Xaa Xaa Xaa Xaa Xaa Xaa Xaa Xaa Xaa Xaa Trp Xaa Xaa Xaa Xaa
           35                  40                  45
   Xaa Xaa Xaa
       50
<210> 4
   <211> 5
   <212> PRT
   <213> Zea mays
<220>
   <221> VARIANT
   <222> (4)..(4)
   <223> /Replace = Glu
<400> 4
   Leu Asn Leu Asp Leu
   1               5
<210> 5
   <211> 828
   <212> DNA
   <213> Artificial
<220>
   <223> séquence synthétique codant ZmMYB31
<400> 5
   atgggtagaa gtccctgctg cgaaaaggca catactaata aaggagcgtg gacgaaggaa      60
   gaggacgaga gactggtggc acatattcgg gcgcacggcg aaggatgctg gcgctccctc     120
   cctaaggccg ctggtttgct gcggtgcggc aagtcttgcc gcctccggtg gatcaactac     180
   ttgaggccgg acttgaagcg cggtaatttc acggaagagg aggacgagct tatcgtcaag     240
   cttcacagtg tcctcggtaa taagtggtct ttgatcgccg gcaggctgcc aggtaggact     300
   gataacgaga taaagaatta ctggaatacc catatccgca gaaaattgct tagcagaggc     360
   atagatcccg tgacgcatag gccagtcaca gaacaccacg cctctaacat caccatctct     420
   ttcgagacag aggtggccgc agcggcccgt gatgacaaga agggcgctgt gttccggctt     480
   gaggacgagg aggaggaaga gagaaataag gccactatgg tggtgggtcg ggatagacaa     540
   tcccagagcc atagccattc gcaccctgct ggtgaatggg gccaaggaaa gcgccctctg     600
   aagtgcccgg acctgaacct ggacttgtgt attagcccac cctgccaaga agaggaggag     660
   atggaggagg ccgccatgcg tgtcaggcca gctgtgaagc gcgaagcggg cctgtgcttt     720
   ggctgctcgc tcggcctgcc aaggaccgcc gattgcaagt gctccagtag cagcttcctc     780
   ggcctgagaa ccgcgatgct cgatttccgg agcctggaga tgaaatag                  828

## Description

La présente invention concerne un procédé d'obtention de plantes tolérantes à un déficit hydrique.

Le « déficit hydrique » correspond à une situation où la quantité d'eau transpirée par une plante est supérieure à la quantité d'eau absorbée par ladite plante.

Le déficit hydrique est l'un des stress abiotiques les plus importants pour les plantes. Il peut affecter leur croissance et leur reproduction, conduisant ainsi à une perte de rendement.

Par conséquent, il est important d'identifier des gènes qui possèdent la capacité d'améliorer la tolérance des plantes au déficit hydrique.

La famille des facteurs de transcription R2R3-MYB *(« myeoblastosis oncogene* ») compte 126 membres chez *Arabidopsis thaliana* (STRACKE et al., Curr. Opin. Plant Biol., 4:447-546, 2001), 84 membres chez le riz (JIANG et al., Genome Bio., 5:R46, 2004) et 192 membres chez le peuplier (WILKINS et al., Plant Physiol., 149:981-993, 2009). Cette famille est caractérisée par la présence des motifs R2 (de séquence X₅WX₁₉WX₁₉WX₇, SEQ ID NO : 2) et R3 (de séquence X₂₄WX₁₈WX₇, SEQ ID NO : 3) de liaison à l'ADN qui régulent de nombreux processus physiologiques, dont le contrôle du cycle cellulaire (STRACKE *et al.,* 2001, cité ci-dessus, FORNALE et al., Plant Mol. Biol., 62:809-823, 2006). L'alignement des séquences peptidiques déduites des séquences nucléotidiques codant ces facteurs de transcription a permis de les classer en différentes sous-familles (STRACKE *et al.,* 2001, cité ci-dessus). Les membres d'une même sous-famille - qui possèdent des sites similaires de liaison à l'ADN et d'interaction protéine-protéine - partagent en partie des fonctions biologiques communes (JIN and MARTIN, Plant. Mol. Biol., 41:577-585, 1999).

Les facteurs de transcription R2R3-MYB de la sous-famille 4 (FORNALE *et al.,* 2006, cité ci-dessus) sont généralement impliqués dans le métabolisme des phénylpropanoïdes et la biosynthèse de la lignine (JIN et al., EMBO J., 19:6150-6561, 2000). Ces facteurs de transcription possèdent, outre les deux motifs peptidiques R2 et R3, le motif peptidique de séquence LNL[D/E]L ; SEQ ID NO : 4) dans la partie C-terminale de la protéine (STRACKE *et al.,* 2001, cité ci-dessus).

Les facteurs de transcription R2R3-MYB de la sous-famille 4 sont bien connus de l'homme du métier. A titre d'exemple de facteurs de transcription R2R3-MYB de la sous-famille 4, on peut citer ceux identifiés par FORNALE et *al.,* 2006 et WILKINS *et al.,* 2009 (cités ci-dessus) et décrits dans le Tableau I ci-après:

| **Espèce** | **Nom de la protéine** | **Numéro d'accession dans les bases de données GENBANK ou UniProtKB** | **Référence : (a) : FORNALE *et al.,* 2006 (b) : WILKINS *et al.,* 2009** |
|---|---|---|---|
| *Arabidopsis thaliana* | AtMYB7 | U26937 | (a) et (b) |
| | AtMYB32 | NM_119665 | |
| | AtMYB4 | AY519615 | |
| | AtY49 | CAA62033 | |
| | AtMYB3 | AF062859 | |
| | AtMYB8 | NP849749 | |
| | AtMYB6 | AL161515 | |
| | AtCAB78069 | CAB78069 | |
| *Populus trichocarpa* | PtrMYB156 | | (b) |
| | PtrMYB221 | | |
| *Populus tremula x Populus tremuloides* | PttMYB | CAD98762 | (a) |
| *Vitis vinifera* | Vv3g1569838 | | (b) |
| | Vv4g15106462 | | |
| *Zea mays* | ZmMYB38 | P20025 | (a) et (b) |
| | ZmMYB31 | AM156906 | |
| | ZmMYB42 | AM156908 | |
| | ZmMYB8 | AM156905 | |
| *Solanum lycopersicum* | S1MYB27 (ou LeMYB27) | CAA64614 | (a) et (b) |
| *Eucalyptus gunii* | EgMYB1 | CAE09058 | (a) et (b) |
| *Picea glauca* | PgMYB5 | | (b) |
| | PgMYB10 | | |
| | PgMYB13 | | |
| | PtMYB14 | | |
| *Tradescantia fluminensis* | TfMYB2 | AAS19476 | (a) |
| | TfMYB6 | AAS19480 | |
| | TfMYB1 | AAS19475 | |
| *Oryza sativa* | OsNP91576 | NP_91576 | (a) |
| | OsT02984 | T02984 | |
| | OsAAV59423 | AAV59423 | |
| | OsPTYPE2 | XP_483665 | |
| | OsPTYPE1 | AAL84628 | |
| *Hordeum vulgare* | HvMYB5 | CAA50221 | (a) et (b) |
| | HvMYB1 | CAA50224 | |
| *Triticum aestivum* | TaMYB1 | AAT37167 | (a) |
| *Dendrobium sp.* | DspMYB8 | AAO49417 | (a) |
| | DspMYB10 | AAO49419 | |
| *Gossypium hirsutum* | GhMYB9 | AAK19619 | (a) |
| | GhMYB1 | AAN28270 | |
| *Antirrhinum majus* | AmMYB308 | JQ0960 | (a) |
| | AmMYB330 | JQ0957 | |

Bien qu'appartenant à la même sous-famille, les facteurs de transcription R2R3-MYB de la sous-famille 4 possèdent des fonctions différentes. En effet :
- des plantes de tabac transformées avec la séquence codante des gènes *d'Antirhinum majus AmMYB308* ou *AmMYB330* présentent un phénotype de réduction de la taille et de la longévité des feuilles exposées à la lumière dont la sévérité est corrélée au niveau d'expression du facteur de transcription (TAMAGNONE et al., Plant Cell, 10:135-154, 1998). Cependant, chez ces plantes de tabac transformées, la sur-expression *d'AmMYB308* conduit à une répression de l'expression des gènes *C4H (cinnamate 4-hydroxylase), 4CL (4-coumarate-CoA ligase), CAD* (*cinnamyle alcool déshydrogénase*) et *CHS* (*chalcone synthase),* mais n'a aucun effet sur l'expression du gène *PAL (phénylalanine ammonia-lyase),* alors que la sur-expression *d'AmMYB330* conduit aussi à une répression de l'expression du gène *4CL* (*4-coumarate-CoA ligase)* mais n'a aucun effet sur l'expression du gène *CHS* (contrairement aux plantes sur-exprimant *AmMYB308*).
- la sur-expression de la séquence codante du gène *AtMYB4* chez le tabac conduit à une répression de l'expression des gènes *C4H, 4CL* et *CAD.* La sur-expression de la séquence codante de ce gène *AtMYB4* chez *Arabidopsis thaliana* conduit à une répression de l'expression des gènes *C4H, 4CL1 (4-coumarate-CoA ligase* 1) et *4CL3 (4-coumarate-CoA ligase 3), à* une induction de l'expression du gène *CCoAOMT (cafféoyl-CoA o-méthyltransférase),* et est sans effet sur l'expression des gènes *PAL2 (phénylalanine ammonia-lyase 2), F5H* (*férulate-5-hydroxylase), COMT (acide caféique o-méthyltransférase)* et *CAD1 (cinnamyle alcool déshydrogénase 1*) (JIN et *al.,* 2000, cité ci-dessus).
- la sur-expression de la séquence codante du gène *ZmMYB31* chez *Arabidopsis thaliana* conduit à une répression de l'expression des gènes *C3H* (*4-coumarate 3-hydroxylase*), *4CL1, F5H* et *COMT,* à une induction de l'expression des gènes *CHI (chalcone isomérase), F3H (flavone 3-hydroxylase), F3'H* (*flavonoïde 3'-hydroxylase)* et *DFR (dihydroflavonol réductase)* et est sans effet sur l'expression des gènes *PAL1 (phénylalanine ammonia-lyase 1*), *PAL2, C4H, HCT (hydroxycinnamoyle-CoA shikimate* / *quinate hydroxycinnamoyle transférase*), *4CL2* (*4-coumarate-CoA ligase 2), CCoAOMT, CCR (cinnamoyle-CoA réductase), CAD, Actin, CHS, FLS* (flavonol synthase)et *UGT73B2* (*UDP sucre glycosyltransférase).* Cette surexpression conduit aussi à une augmentation des sous-unités H (p-hydroxyphényle) de la lignine dans ces plantes transgéniques (Fornalé et al., The Plant Journal, 64, 633-644, 2010).
- la sur-expression de la séquence codante du gène *ZmMYB42* (dont la protéine codée présente 62,1% d'identité et 70,0% de similarité avec la séquence peptidique de ZmMYB31) chez *Arabidopsis thaliana* conduit à une répression de l'expression des gènes *PAL1, CQH, F5H, 4CL1, HCT, COMT, ALDH (aldéhyde déshydrogénase), CAD, F3H* et *F3'H,* alors qu'elle induit l'expression du gène *CHS,* et est sans effet sur l'expression des gènes *CHI, FLS, UGTs* (*UDP sucre glycosyltransférase), SGT (sinapate sinapoyle transférase)* et *SMT (sinapoyle-glucose malate sinapoyle transférase*). Cette surexpression conduit aussi à une diminution des sous-unités S (syringyle) de la ligne et à une augmentation des sous-unités H (p-hydroxyphényle) et G (guaiacyle) de la lignine dans ces plantes transgéniques (SONBOL et al., Plant Mol. Biol., 70:283-96, 2009).

La Demande Internationale WO 01/32002 décrit une méthode pour augmenter la tolérance d'une plante à un stress abiotique (par exemple sécheresse, température, salinité), comprenant la modification du génome de ladite plante pour surexprimer dans ladite plante un facteur de transcription MYB choisi parmi les facteurs de transcription MYB60 (appartenant à la sous-famille 1 selon la définition des sous-famille donnée par STRACKE *et al.,* 2001, cité ci-dessus), MYB74 (appartenant à la sous-famille 11), MYB75 (appartenant à la sous-famille 6) et MYB90 (appartenant aussi à la sous-famille 6) d'*A. thaliana.*

La Demande Internationale WO 2009/056566 décrit un procédé pour augmenter, chez une plante, des caractères liés au rendement (telles que la biomasse) en modulant l'expression dans ladite plante d'un facteur de transcription MYB7. Cette augmentation des caractères liés au rendement peut s'effectuer dans des conditions de stress biotique ou abiotique. Plusieurs séquences polypeptidiques de maïs décrites comme étant des facteurs de transcription MYB7 sont divulguées dans ce document. Ces facteurs de transcription « MYB7 » englobent, chez le maïs, le facteur de transcription ZmMYB31 (identifié en tant que séquence SEQ ID NO : 83 dans ce document) ainsi que plusieurs séquences polypeptidiques présentant au moins 47% d'identité avec la séquence polypeptidique de ZmMYB31. Cependant, ce document ne montre pas qu'une surexpression d'un facteur de transcription de maïs « MYB7 » chez une plante augmente la tolérance d'une plante à un déficit hydrique. En outre, dans la mesure où il existe des différences significatives de fonction entre les différents facteurs de transcription R2R3-MYB d'une même sous-famille, il est peu probable que la surexpression, chez une plante, de chacun des facteurs de transcription « MYB7 » décrits dans ce document permette d'augmenter des caractères liés au rendement quelles que soient les conditions de stress biotique ou abiotique, notamment en condition de déficit hydrique.

Au cours de leurs travaux, les inventeurs ont mis en évidence que des plantes de maïs (*Zea mays*) transgéniques surexprimant le facteur de transcription ZmMYB31 présentaient une tolérance augmentée à un déficit hydrique par rapport aux plantes de maïs sauvages (non transgéniques). Le facteur de transcription ZmMYB31 de maïs (disponible dans la base de données GENBANK sous le numéro d'accession GI:89143144 ; référencée également sur la puce du Maize Oligonucleotide Array Project [http://www.maizearray.org] sous le numéro MZ00022562) appartient à la sous-famille 4 des facteurs de transcription R2R3-MYB. Il est également représenté par la séquence SEQ ID NO : 1.

Par ailleurs, les inventeurs ont également recherché, chez le maïs (non transgénique), les gènes candidats associés à une région du maïs localisée sur le chromosome 2 et contenant un QTL (locus de caractères quantitatifs) de sensibilité de la croissance foliaire au déficit hydrique édaphique, ainsi qu'un QTL de protandrie en condition de sécheresse (WELCKER et al., J Exp Bot., 58, 339-349, 2007). Ils ont alors identifié le gène codant le facteur de transcription R2R3-MYB de la sous-famille 4 ZmMYB31 qui co-localise avec la région visée et dont l'abondance relative en transcrits est régulée par le déficit hydrique et varie entre deux sous-populations de lignées recombinantes de maïs différant génétiquement pour la région ciblée et hétérogène sur le reste du génome. De manière inattendue, aucun autre gène codant pour un facteur de transcription R2R3-MYB de la sous-famille 4, tel que ceux décrits dans le tableau I ci-dessus, n'a pu être identifié par cette analyse (combinant l'analyse du niveau d'expression des gènes entre deux sous-populations de lignées recombinantes de maïs différant génétiquement pour la région ciblée et hétérogène sur le reste du génome, et la cartographie).

La présente invention propose en conséquence d'utiliser la protéine ZmMY31 pour augmenter la résistance des plantes au déficit hydrique.

La présente invention a pour objet un procédé pour augmenter la tolérance d'une plante au déficit hydrique, caractérisé en ce que l'on surexprime dans ladite plante un facteur de transcription R2R3-MYB de la sous-famille 4, possédant au moins 95% d'identité, et par ordre de préférence croissant au moins 96%, 97%, 98% et 99% d'identité, avec la séquence SEQ ID NO: 1.

Sauf précision contraire, l'alignement entre deux séquences peptidiques et le calcul des pourcentages d'identité sont effectués sur toute la longueur des séquences peptidiques à l'aide du programme d'ordinateur « *needle* » (NEEDLEMAN et WUNSCH, J. Mol. Biol., 48, 443-453, 1970) en utilisant les paramètres par défaut : « *Matrix* » : EBLOSUM62, « *Gap penalty* » : 10,0 et « *Extend penalty* » : 0,5.

On entend par un facteur de transcription R2R3-MYB de la sous-famille 4, un facteur de transcription R2R3-MYB tel que décrit par STRACKE *et al.,* 2001 (cité ci-dessus), possédant les motifs conservés R2 (de séquence X₅WX₁₉WX₁₉WX₇, SEQ ID NO : 2) et R3 (de séquence X₂₄WX₁₈WX₇, SEQ ID NO : 3) de liaison à l'ADN, et le motif conservé LNL[E/D]L (SEQ ID NO : 4).

Selon un mode de réalisation avantageux de la présente invention, ledit facteur de transcription R2R3-MYB de la sous-famille 4 est issu d'une plante monocotylédone et sa séquence peptidique comprend les acides aminés conservés situés aux positions 1-9, 11-13, 15-22, 24-25, 27, 30-41, 43-70, 74-75, 77-78, 80-83, 85-93, 95-111, 113-116, 120-127, 138, 140-141, 197, 202-212, 214, 234, 239, 242, 252, 254-255, 261-263, 267-271 et 274-275 de ladite séquence SEQ ID NO : 1 lorsqu'elle est alignée avec ladite séquence SEQ ID NO : 1. Ces acides aminés conservés ont été déterminés par les Inventeurs en comparant la séquence peptidique des paralogues et des orthologues chez les plantes monocotylédones *H. vulgare, O*. *sativa* et *T. aestivum,* avec la séquence peptidique du facteur de transcription ZmMYB31.

On entend par un facteur de transcription R2R3-MYB de la sous-famille 4 issu d'une plante monocotylédone, un facteur de transcription R2R3-MYB de la sous-famille 4 exprimée par une plante monocotylédone ou un facteur de transcription R2R3-MYB de la sous-famille 4 synthétique obtenu par mutation d'un facteur de transcription R2R3-MYB de la sous-famille 4 exprimé par une plante monocotylédone.

La présente invention s'applique à toutes les plantes monocotylédones ou dicotylédones, et notamment aux plantes sensibles au déficit hydrique. De manière non limitative, elle peut s'appliquer aux plantes potagères, aux plantes ornementales, aux arbres fruitiers, aux plantes de grandes cultures telles que le blé, le maïs ou le riz, ou aux plantes de cultures industrielles comme le cotonnier, le colza ou le tournesol, de préférence le maïs.

La surexpression (augmentation de l'expression) dans une plante d'un facteur de transcription R2R3-MYB de la sous-famille 4 tel que défini ci-dessus, peut être effectuée par la modification du génome de ladite plante. Cette modification du génome peut notamment s'effectuer par transformation génétique de ladite plante par une ou plusieurs copies d'un polynucléotide codant pour ledit facteur de transcription de la sous-famille 4, associé à des séquences de régulation en *cis* de son expression. La surexpression dudit facteur de transcription R2R3-MYB de la sous-famille 4 peut également être obtenue par modification des séquences de régulation en *cis* de l'expression dudit facteur de transcription R2R3-MYB de la sous-famille 4, par exemple en remplaçant son promoteur endogène par un promoteur plus fort, permettant un niveau de transcription plus élevé, ou bien en adjoignant au promoteur endogène des séquences activatrices de la transcription, de type « amplificateur », ou de la traduction.

Pour la mise en oeuvre du procédé selon la présente invention, on utilise une cassette d'expression recombinante, comprenant un polynucléotide codant pour un facteur de transcription R2R3-MYB de la sous-famille 4 tel que défini ci-dessus, placé sous le contrôle transcriptionnel d'un promoteur approprié.

Ledit promoteur peut être un promoteur hétérologue. Dans ce cas, on peut utiliser par exemple :
- des promoteurs constitutifs, tels que le promoteur de la gluténine de haut poids moléculaire spécifique de l'endosperme (VERDAGUER et al., Plant Mol Biol., 31:1129-1139, 1996), le promoteur de l'ARN 35S (ODELL et al., Nature, 313:810-812, 1985) ou 19S (KAY et al., Science, 236:1299-1302, 1987) du CaMV, le promoteur de l'actine 1 du riz (McELROY et al., Plant Cell, 2:163-171, 1990), le promoteur de l'ubiquitine 3 du riz ou du maïs (SIVAMANI et QU, Plant Mol Biol., 60:225-239, 2006),
- des promoteurs spécifiques du phloème, tels que le promoteur du Wheat Dwarf Virus (DINANT et al., Physiologia plantarum 121 : 108-116, 2004 ; Demande PCT WO 03/060135) ou le promoteur de *AtPP2-A1* (DINANT et al., Plant Physiol., 131 : 114-128, 2003),
- des promoteurs spécifiques des feuilles, tels que le promoteur de la petite sous-unité de la Rubisco, le promoteur de la phosphoénolpyruvate carboxylase,
- des promoteurs spécifiques des racines, tels que le promoteur RCc3 du riz (Demande Internationale WO 2009/016104), le promoteur antiquitine du riz (Demande Internationale WO 2007/076115), ou
- des promoteurs localement inductibles par le stress (sécheresse, salinité), tels que le promoteur rd29 *d'Arabidopsis* (YAMAGUCHI-SHINOZAKI et SHINOZAKI, Mol. Gen. Genet., 236: 331-340, 1993),
de préférence le promoteur de la gluténine de haut poids moléculaire spécifique de l'endosperme.

On peut également utiliser le promoteur d'un facteur de transcription R2R3-MYB d'une autre sous-famille que la sous-famille 4.

Pour la mise en oeuvre du procédé selon la présente invention, on peut aussi utiliser des vecteurs recombinants, résultant de l'insertion d'une cassette d'expression telle que décrite ci-dessus dans un vecteur hôte.

Les cassettes d'expression et vecteurs recombinants tels que décrits ci-dessus peuvent, bien entendu, comprendre en outre d'autres séquences, usuellement employées dans ce type de constructions. Le choix de ces autres séquences sera effectué, de manière classique, par l'homme du métier en fonction notamment de critères tels que les cellules-hôtes choisies, les protocoles de transformation envisagés, etc.

On citera, à titre d'exemples non limitatifs, les terminateurs de transcription, les séquences de tête (séquences « *leader* ») et les sites de polyadénylation. Ces séquences peuvent être celles qui sont naturellement associées au gène codant le facteur de transcription R2R3-MYB de la sous-famille 4 tel que défini ci-dessus, ou bien peuvent être des séquences hétérologues. Ces séquences n'interviennent pas sur les propriétés spécifiques du promoteur ou du gène auxquelles elles sont associées, mais peuvent améliorer globalement qualitativement ou quantitativement, la transcription, et le cas échéant, la traduction. A titre d'exemples de séquences de ce type fréquemment utilisées chez les plantes, on citera parmi les plus répandues, le terminateur de l'ARN 35S du CaMV et le terminateur du gène de la nopaline synthase. On peut également, dans le but d'augmenter le niveau d'expression, utiliser des séquences amplificatrices (séquences « *enhancer* » de transcription et de traduction).

Parmi les autres séquences couramment employées dans la construction de cassettes d'expression et vecteurs recombinants, on citera également les séquences permettant le suivi de la transformation, et l'identification et/ou la sélection des cellules ou organismes transformés. Il s'agit notamment de gènes rapporteurs, conférant aux cellules ou organismes transformés un phénotype aisément reconnaissable, ou bien de gènes marqueurs de sélection : seules les cellules ou organismes exprimant un gène marqueur de sélection déterminé, sont viables dans des conditions données (conditions sélectives). Des gènes rapporteurs fréquemment employés sont par exemple celui de la beta-glucuronidase (GUS), celui de la luciférase, ou celui de la « *green fluorescent protein »* (GFP). Des gènes marqueurs de sélection sont généralement des gènes de résistance à un antibiotique, ou également, dans le cas des plantes ou des cellules végétales, à un herbicide. Il existe une très grande variété de gènes marqueurs de sélection parmi lesquels l'homme du métier peut effectuer son choix en fonction des critères qu'il aura lui-même déterminés.

Pour la mise en oeuvre du procédé selon la présente invention, on peut également utiliser des cellules-hôtes transformées par un polynucléotide codant pour un facteur de transcription R2R3-MYB de la sous-famille 4 tel que défini ci-dessus, ce qui inclut en particulier les cellules hôtes transformées par une cassette d'expression ou un vecteur recombinants tels que décrits ci-dessus.

On entend par cellule ou organisme transformé par un polynucléotide, toute cellule ou organisme dont le contenu génétique a été modifié par transfert dudit polynucléotide dans ladite cellule ou ledit organisme, quelle que soit la méthode de transfert qui a été utilisée, et que l'information génétique apportée par ledit polynucléotide soit intégrée dans l'ADN chromosomique ou demeure extra-chromosomique.

Les cellules hôtes peuvent être des cellules procaryotes ou eucaryotes. Dans le cas de cellules procaryotes, il peut notamment s'agir d'Agrobactéries telles *qu'Agrobacterium tumefaciens ou Agrobacterium rhizobium.* Dans le cas de cellules eucaryotes, il peut s'agir notamment de cellules végétales, issues de plantes monocotylédones ou dicotylédones.

Les plantes transgéniques peuvent être obtenues par transformation génétique par au moins un polynucléotide, une cassette d'expression ou un vecteur recombinant tels que définis ci-dessus.

Lesdites plantes transgéniques englobent les plantes monocotylédones transgéniques, de préférence une plante de maïs transgénique, comprenant au moins un transgène contenant une cassette d'expression recombinante comprenant un polynucléotide codant pour un facteur de transcription R2R3-MYB de la sous-famille 4 tel que défini ci-dessus.

On définit ici comme plante transgénique une plante transformée chez laquelle l'information génétique exogène apportée par un polynucléotide transformant est intégrée de manière stable dans l'ADN chromosomique, sous forme de transgène, et peut ainsi être transmise aux descendants de ladite plante. Cette définition englobe donc également les descendants des plantes résultant de la transgénèse initiale, dès lors qu'ils contiennent dans leur génome une copie du transgène.

Différentes méthodes d'obtention de plantes transgéniques sont bien connues en elles-mêmes de l'homme du métier. Généralement, ces méthodes impliquent la transformation de cellules végétales, la régénération de plantes à partir des cellules transformées, et la sélection des plantes ayant intégré le transgène.

De nombreuses techniques de transformation de cellules végétales germinales ou somatiques (isolées, sous forme de cultures de tissus ou d'organe, ou sur la plante entière), et de régénération des plantes sont disponibles. Le choix de la méthode la plus appropriée dépend généralement de la plante concernée.

A titre d'exemples non limitatifs de méthodes utilisables dans le cas des plantes mentionnées ci-dessus, il est possible de citer les protocoles décrits par GUIS et al. (Scientia Horticulturae 84 : 91-99, 2000) pour le melon, par HAMZA et CHUPEAU (J. Exp. Bot. 44 : 1837-1845, 1993) pour la tomate, par SHOEMAKER et al. (Plant Cell Rep. 3 : 178-181, 1986), par TROLINDER et GOODIN (Plant Cell Rep. 6 : 231-234, 1987) pour le cotonnier, par VAN DER MARK et al. (J. Genet Breeding 44 : 263-268, 1990) ou par MARCHANT et al. (Ann. Bot. 81 : 109-114, 1998) pour les rosiers. Dans le cas des plantes monocotylédones, on peut citer par exemple les protocoles décrits par HIEI et al. (The Plant Journal, 6, 271-282, 1994) ou ISHIDA et al. (Nature biotechnology, 14, 745-750, 1996) pour le maïs, ou par RASCO-GAUNT et al. (J. Exp. Bot. 52 : 865-874, 2001) pour le blé.

A titre d'exemple complémentaire, l'obtention d'A. *thaliana* surexprimant le facteur de transcription ZmMYB31 a été décrite par FORNALE *et al.,* 2006 (cité ci-dessus).

La présente invention a aussi pour objet l'utilisation d'un polynucléotide isolé codant un facteur de transcription R2R3-MYB de la sous-famille 4 tel que défini ci-dessus, de préférence le facteur de transcription ZmMYB31 de séquence SEQ ID NO : 1, pour induire chez une plante une tolérance à un stress hydrique.

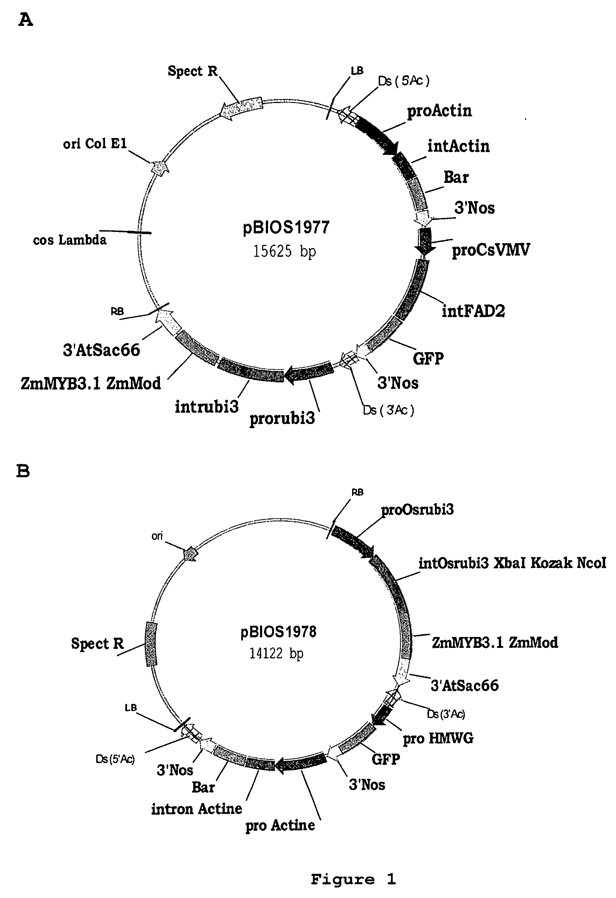

La présente invention sera mieux comprise à l'aide du complément de description qui va suivre, qui se réfère à des exemples non-limitatifs illustrant l'obtention de plantes transgéniques surexprimant le facteur de transcription R2R3-MYB de la sous-famille 4 ZmMYB31 et la mise en évidence de son rôle dans l'augmentation de la résistance au déficit hydrique, ainsi que de la figure 1 annexée représentant la carte des vecteurs binaires pBIOS1977 (A) et pBIOS1978 (B).

### EXEMPLE 1 : OBTENTION DE MAÏS TRANSGENIQUES SUREXPRIMANT LE FACTEUR DE TRANSCRIPTION ZMMYB31

### 1) Clonage et transformation génétique du maïs

Deux vecteurs de transformation différents (pBIOS 1562 et pBIOS 1958) ont été utilisés pour la transformation génétique du maïs. Ces vecteurs contiennent le gène bar de *Streptomyces hygroscopicus* conférant la résistance à l'herbicide bialaphos (WHITE et al., Nucleic Acids Res., 18:1062, 1990), utile pour la sélection des transformants de maïs, et un gène codant une GFP *(Green Fluorescente Protein)* en tant que marqueur visuel pour suivre la présence du transgène dans les plantes et les graines. La différence entre ces deux vecteurs réside dans la stratégie de clonage utilisée pour introduire la cassette d'expression contenant le gène d'intérêt (clonage via le système Gateway® ou clonage par restriction) et le promoteur pour l'expression de la GFP (le promoteur du virus de la mosaïque des nervures du manioc (CsVMV) suivi par l'intron FAD2 *d'Arabidopsis* ou le promoteur de la gluténine de haut poids moléculaire spécifique de l'endosperme).

Selon une première stratégie de clonage, le gène synthétique codant ZmMYB31 (SEQ ID NO : 5 ; séquence synthétique optimisée pour l'expression dans le maïs) contenant les sites de restriction *att*L1 et *att*L2 a été introduit par une réaction de recombinaison LR dans le vecteur binaire de destination GATEWAY pBIOS 1562, générant ainsi le vecteur pBIOS1977 (voir Figure 2A). Le vecteur pBIOS 1562 est dérivé du vecteur pSB12 (KOMARI et al., Plant J., 10:165-174, 1996) contenant le gène bar sous le contrôle du promoteur pActin, le gène codant une GFP sous le contrôle du promoteur du CsVMV suivi par l'intron FAD2, le promoteur et le 1^{er} intron de l'ubiquitine 3 du riz (SIVAMANI et QU, Plant Mol Biol., 60:225-239, 2006) suivie d'une cassette GATEWAY et d'une séquence de polyadénylation provenant du gène Sac66 d'*Arabidopsis* (JENKINS et al., Plant Cell Environ., 22:159-167, 1999).

Selon une seconde stratégie de clonage, le gène synthétique codant ZmMYB31 (SEQ ID NO : 5) a été introduit par clonage par restriction (présence de sites de restriction *SapI* entre la région codante et les sites *att*L) dans le vecteur binaire de destination pBIOS 1958 digéré par *SapI,* générant ainsi le vecteur pBIOS1978 (voir Figure 2B). pBIOS 1958 est également dérivé du vecteur pSB12, mais possède le gène codant une GFP sous le contrôle du promoteur de la gluténine de haut poids moléculaire spécifique de l'endosperme (promoteur HMWG).

les vecteurs pBIOS1977 ou pBIOS1978 ont ensuite été transférés dans la souche *d'Agrobacterium tumefaciens* LBA4404 (pSB1) selon la méthode décrite par KOMARI *et al.,* 1996 (cité ci-dessus).

Le cultivar de maïs A188 a ensuite été transformé par cette souche d'agrobactérie contenant le vecteur pBIOS1977 ou le vecteur pBIOS1978, selon la méthode décrite par ISHIDA *et al.,* 1996 (cité ci-dessus).

Les transformants primaires (T0) ont été sélectionnés selon des méthodes de routine en fonction des quatre critères suivantes :
(i) *nombre de copies insérées* : Cette détermination a été réalisée par PCR quantitative. Tous les événements de transformation possédant plus de 2 copies du transgène ont été éliminés.
(ii) *intégrité de l'ADN-T inséré :* Elle a été vérifiée par une réaction de PCR au cours des premières étapes de développement de la plante transformée.
(iii) *absence de terminaison prématurée de la transcription du transgène* : Chacun des gènes ciblés étant sous le contrôle d'un promoteur constitutif, il est possible de mesurer leur expression à partir de tissus foliaires. Les ARN de feuilles de plantes T0 ont donc été extraits et l'intégrité des transcrits vérifiée par RT-PCR à l'aide d'une amorce sens située sur l'intron ubiquitine3 de riz et d'une amorce antisens située sur le terminateur AtSac66.
(iv) *nombre de gra ins T1 récoltés.*

Après sélection des transformants, il a été obtenu 52 lignées trangéniques dont 21 ont un transgène unique et intègre.

### 2) Evaluation de la tolérance des plantes transgéniques au déficit hydrique

Des plantes de première génération (croisement du transformant primaire avec la lignée récurrente A188) sont évaluées sur une plate-forme de phénotypage. Ces plantes transgéniques sont donc hémizygotes pour le transgène (caractère dominant de la transformation génétique). Les témoins (« RRS » et « RCP) utilisés dans l'expérimentation correspondent aux ségrégants sauvages issus de ce même croisement.

### 2.1 Compartiment de culture

Les plantes étudiées sont cultivées en phytotron. Ce dernier, d'une surface de 30 m² dispose de deux chambres de culture indépendantes. Dans ces chambres l'éclairage, la température et l'hygrométrie sont régulés (voir paragraphe 2.2. ci-dessous).

Les semis sont réalisés dans des terrines de dimensions 44 x 28,5 x 7 cm (H x 1 x L). Cinq génotypes sont semés par terrine à raison de dix graines par génotype. Cinq plantes seulement par génotype sont utilisées pour mesurer la cinétique de dessèchement.

### 2.2 Conditions de culture

Au sein du compartiment de culture la température, l'humidité et l'éclairage sont régulés.

Les conditions appliquées sont les suivantes :

### Photopériode :

- Jour durant 16h (6h à 22h) avec complément photosynthétique (lampe à sodium 400W) lorsque le rayonnement extérieur est inférieur à 100W/m².
- Nuit durant 8h (22h à 6h).

### Thermopériode : 24°C / 20°C.

Le respect de ces conditions est assuré par un chauffage lorsque la température est inférieure à 20°C la nuit ou 24°C le jour, lorsque la température dépasse 25°C.

*Humidité* : 75% d'humidité relative régulée par brumisation nocturne.

Ces différentes conditions assurent une croissance optimale du maïs.

### 2.3 Mesure de la cinétique de dessèchement

### Pertinence du caractère mesuré :

Le comportement des plantes vis-à-vis de la transpiration est étudié grâce au suivi en continu de la baisse de la teneur en eau relative (RWC : Relative Water Content) de plantules à un stade jeune (4 feuilles visibles). L'objectif est d'étudier la réponse en termes de contrôle stomatique des plantes lors d'un arrêt brutal de l'alimentation en eau.

Un contrôle stomatique très rapide à l'apparition d'un déficit hydrique permet d'économiser l'eau disponible mais limite la capacité d'assimilation du CO₂ donc le potentiel de production de la plante. En revanche, une fermeture assez tardive des stomates permet le maintien de l'activité photosynthétique de la plante assurant le maintien du potentiel de production au risque de se dessécher plus rapidement (Khalfaoui, 1991, In : L'amélioration des plantes pour l'adaptation aux milieux arides. Ed. AUPELF-UREF. John Libbey Eurotext, pp. 51-63).

### Méthode :

Les mesures sont réalisées sur des plantules T1 entières au stade 3-4 feuilles visibles. Les plantes utilisées au cours de cette mesure sont des plantes issues d'un semis en excès par rapport aux besoins de la plate-forme (3 graines semées par pot). Les effectifs pour la mesure de dessèchement sont de 5 plantes par évènement de transformation et témoins sauvages.

Les plantes sont coupées au niveau du collet, immergées pendant 24 heures à 4°C à l'obscurité (pour saturer les cellules en eau) puis placées dans une enceinte climatique lumineuse régulée à 30°C.

Un suivi du poids des plantules est alors réalisé selon le calendrier détaillé dans le Tableau II ci-dessous :

Tableau II : Calendrier des pesées des plantules conditionnées à 30°C en pleine lumière. Le poids à H0 correspond au poids à pleine turgescence. En fin de jour 3, les plantules sont placées dans une étuve à 80°C pendant 24h00 afin d'obtenir par une dernière pesée la valeur de poids sec.

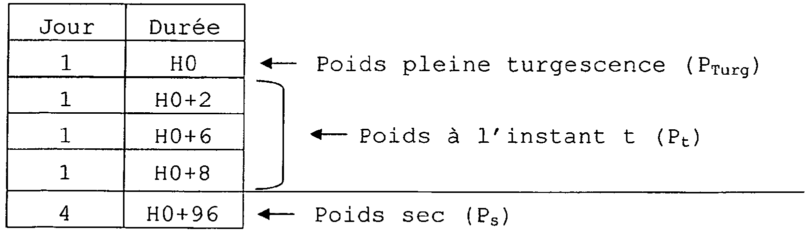

A l'instant t, la teneur en eau relative des plantes est ensuite calculée selon la formule mathématique suivante : (Pₜ-Pₛ)/(P_{Turg}-Pₛ) x 100.

## Revendications

1. Procédé pour augmenter la tolérance d'une plante au déficit hydrique, **caractérisé en ce que** l'on surexprime dans ladite plante un facteur de transcription R2R3-MYB de la sous-famille 4 possédant au moins 95% d'identité avec la séquence SEQ ID NO: 1.

2. Procédé selon la revendication 1, **caractérisé en ce que** ledit facteur de transcription R2R3-MYB de la sous-famille 4 est issue d'une plante monocotylédone, et comprend les acides aminés conservés situés aux positions 1-9, 11-13, 15-22, 24-25, 27, 30-41, 43-70, 74-75, 77-78, 80-83, 85-93, 95-111, 113-116, 120-127, 138, 140-141, 197, 202-212, 214, 234, 239, 242, 252, 254-255, 261-263, 267-271 et 274-275 de ladite séquence SEQ ID NO : 1 lorsqu'il est aligné avec ladite séquence SEQ ID NO : 1.

3. Procédé selon la revendication 1 ou la revendication 2, **caractérisé en ce que** ledit facteur de transcription R2R3-MYB de la sous-famille 4 possède la séquence SEQ ID NO : 1.

4. Procédé selon l'une quelconque des revendications 1 à 3, **caractérisé en ce que** ladite plante est choisie parmi les plantes potagères, les plantes ornementales, les arbres fruitiers, le blé, le maïs, le riz, le cotonnier, le colza et le tournesol.

5. Procédé selon la revendication 4, **caractérisé en ce que** ladite plante est le maïs.

6. Procédé selon l'une quelconque des revendications 1 à 5, **caractérisé en ce que** ladite surexpression est obtenue :
- par transformation génétique de ladite plante par une ou plusieurs copies d'un polynucléotide codant pour ledit facteur de transcription, associé à des séquences de régulation en *cis* de son expression, ou
- par modification des séquences de régulation en cis de l'expression dudit facteur de transcription.

7. Utilisation d'un polynucléotide isolé codant un facteur de transcription R2R3-MYB de la sous-famille 4 tel que défini à l'une quelconque des revendications 1 à 3, pour induire chez une plante une tolérance à un stress hydrique.

## Patentansprüche

1. Verfahren zum Erhöhen der Toleranz einer Pflanze gegenüber Wassermangel, **dadurch gekennzeichnet, dass** in der Pflanze ein R2R3-MYB-Transkriptionsfaktor der Unterfamilie 4 überexprimiert wird, der mindestens 95% Identität mit der Sequenz SEQ ID NO: 1 besitzt.

2. Verfahren nach Anspruch 1, **dadurch gekennzeichnet, dass** der R2R3-MYB-Transkriptionsfaktor der Unterfamilie 4 von einer einkeimblättrigen Pflanze stammt und konservierte Aminosäuren umfasst, die sich an den Positionen 1-9, 11-13, 15-22, 24-25, 27, 30-41, 43-70, 74-75, 77-78, 80-83, 85-93, 95-111, 113-116, 120-127, 138, 140-141, 197, 202-212, 214, 234, 239, 242, 252, 254-255, 261-263, 267-271 und 274-275 der Sequenz SEQ ID NO: 1 befinden, wenn sie mit der SEQ ID NO: 1 ausgerichtet ist.

3. Verfahren nach Anspruch 1 oder Anspruch 2, **dadurch gekennzeichnet, dass** der R2R3-MYB-Transkriptionsfaktor der Unterfamilie 4 die Sequenz SEQ ID NO: 1 besitzt.

4. Verfahren nach einem der Ansprüche 1 bis 3, **dadurch gekennzeichnet, dass** die Pflanze unter Gemüsepflanzen, Zierpflanzen, Ostbäumen, Weizen, Mais, Reis, der Baumwollpflanze, Raps und Sonnenblume ausgewählt wird.

5. Verfahren nach Anspruch 4, **dadurch gekennzeichnet, dass** die Pflanze Mais ist.

6. Verfahren nach einem der Ansprüche 1 bis 5, **dadurch gekennzeichnet, dass** das Überexprimieren durch Folgendes erhalten wird:
- durch genetische Transformation der Pflanze durch eine oder mehrere Kopien eines codierenden Polynukleotids für den Transkriptionsfaktor, der mit Regulationssequenzen in cis seiner Expression verknüpft ist, oder
- durch Änderung der Regulationssequenzen in cis der Expression des Transkriptionsfaktors.

7. Verwendung eines isolierten Polynukleotids, das einen R2R3-MYB-Transkriptionsfaktor der Unterfamilie 4 codiert, nach einem der Ansprüche 1 bis 3, um bei einer Pflanze eine Toleranz gegenüber Wasserknappheit zu bewirken.

## Claims

1. A method for increasing the tolerance of a plant to water deficit, **characterized in that** an R2R3-MYB subfamily 4 transcription factor, having at least 95% identity with the sequence SEQ ID NO: 1, is overexpressed in said plant.

2. The method as claimed in claim 1, **characterized in that** said R2R3-MYB subfamily 4 transcription factor is derived from a monocotyledonous plant, and comprises the conserved amino acids located at positions 1-9, 11-13, 15-22, 24-25, 27, 30-41, 43-70, 74-75, 77-78, 80-83, 85-93, 95-111, 113-116, 120-127, 138, 140-141, 197, 202-212, 214, 234, 239, 242, 252, 254-255, 261-263, 267-271 and 274-275 of said sequence SEQ ID NO: 1 when it is aligned with said sequence SEQ ID NO: 1.

3. The method as claimed in claim 1 or claim 2, **characterized in that** said R2R3-MYB subfamily 4 transcription factor has the sequence SEQ ID NO: 1.

4. The method as claimed in any one of claims 1 to 3, **characterized in that** said plant is chosen from edible plants, ornamental plants, fruit trees, wheat, corn, rice, the cotton plant, rape and sunflower.

5. The method as claimed in claim 4, **characterized in that** said plant is corn.

6. The method as claimed in any one of claims 1 to 5, **characterized in that** said overexpression is obtained:
- by genetic transformation of said plant with one or more copies of a polynucleotide encoding said transcription factor, combined with *cis* regulatory sequences for its expression, or
- by modification of the *cis* regulatory sequences for the expression of said transcription factor.

7. The use of an isolated polynucleotide encoding an R2R3-MYB subfamily 4 transcription factor as defined in any one of claims 1 to 3, for inducing water-stress tolerance in a plant.
